Europäisches Patentamt

European Patent Office

Office européen des brevets

(11) Veröffentlichungsnummer: **0 280 062**
**A1**

## EUROPÄISCHE PATENTANMELDUNG

(21) Anmeldenummer: **88101249.6**

(22) Anmeldetag: **28.01.88**

(51) Int. Cl.⁴: **A61N 5/06** , **A47C 27/08**

(30) Priorität: **10.02.87 DE 8701977 U**

(43) Veröffentlichungstag der Anmeldung:
**31.08.88 Patentblatt 88/35**

(34) Benannte Vertragsstaaten:
**AT BE CH ES FR GB IT LI LU NL SE**

(71) Anmelder: **AYK MED. TECHN. GERÄTE GMBH**
**Sülztalstrasse 23**
**D-5063 Overath(DE)**

(72) Erfinder: **Aydnik, Heinrich Karl Martin**
**Am Sonnenhang 26**
**D-5063 Overath 6(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Bräunungsgerät.**

(57) Das Bräunungsgerät weist eine auf einem Sockel (10) angeordnete Liege (11) auf, über der die Strahlungsapparatur (13) angeordnet ist. Die Liege (11) enthält eine Wassermatratze. Die Wassermatratze ermöglicht ein entspannteres Liegen sowie gleichmäßigere Abstände der zu bräunenden Körperteile von der Strahlungsapparatur (13).

FIG.1

EP 0 280 062 A1

## Bräunungsgerät

Die Erfindung betrifft ein Bräunungsgerät nach dem Oberbegriff des Anspruchs 1.

Bekannt sind Hochdruck-Bräunungsgeräte, die eine Liege aufweisen, über der eine Strahlungsapparatur angeordnet ist. Die Strahlungsapparatur liefert ultraviolette Strahlung mit Hochdruck-Quartzlampen und sie kann an einem Ständer vertikal verfahren werden, um auf die jeweils gewünschte Höhe eingestellt zu werden. Die elektrischen Versorgungseinrichtungen zur Erzeugung der Hochspannung für die Strahlungsappartur sowie Steuer- und Regeleinrichtungen sind in einem Sockel der Liege untergebracht. Die Liege weist bei den bekannten Bräunungsgeräten übliche Matratzen auf, z.B. Schaumstoffmatratzen.

Bekannt sind ferner Wasserbetten mit einer wassergefüllten Matratze. Solche Wasserbetten haben den Vorteil, daß sie sich dem Körper anpassen und eine gleich mäßige Verteilung des Auflagedrucks des Körpers bewirken. Solche Wassermatratzen können mit einer elektrischen Heizung ausgestattet sein. Derartige Wasserbetten können als Schlafbetten sowie in ärtztlichen Praxen, für Massage, Gymnastik o.dgl. eingesetzt werden.

Der Erfindung liegt die Aufgabe zugrunde, ein Bräunungsgerät der im Oberbegriff des Anspruchs 1 angegebenen Art zu schaffen, das dem Benutzer ein entspanntes Liegen ermöglicht und das Wohlbefinden während des Bräunungsprozesses steigert sowie ferner den Abstand der Körperteile von der Strahlungsapparatur vergleichmäßigt.

Die Lösung dieser Aufgabe erfolgt erfindungsgemäß mit dem im kennzeichnenden Teil des Anspruchs 1 angegebenen Merkmal.

Dadurch, daß bei dem erfindungsgemäßen Bräunungsgerät die Liege eine Wassermatratze aufweist, erfolgt eine gleichmäßigere Druckverteilung des Körpers des Benutzers. Das Wohlbefinden während des Bräunungsvorgangs wird durch entspanntes Liegen gesteigert. Weiterhin wird durch unterschiedliches Einsinken der Körperteile in die nachgiebige Wassermatratze ein gleichmäßigerer Abstand sämtlicher Körperbereiche von den Bräunungsstrahlers ermöglicht, so daß die Gleichmäßigkeit des Bräunungsvorgangs verbessert wird.

Mit den Merkmalen des Anspruchs 2 wird die Wassermatratze einerseits gegen Beschädigungen geschützt und andererseits wird erreicht, daß auslaufendes Wasser von der Wanne aufgefangen wird.

Die im Anspruch 3 angegebene obere Polsterschicht, die die Wanne bzw. die Wassermatratze bedeckt, wirkt ebenfalls als Schutz gegen Beschädigungen der Wassermatratze, als wärmeisolierende Schicht und als Trennschicht zwischen dem Körper des Benutzers und der die Wassermatratze umgebenden Folie.

Ein Bräunungsgerät, das zur Versorgung, Regelung und Einstellung der Strahlungsapparatur sowie zu deren Bewegung elektrische Komponenten enthält, erfordert einen Schutz der elektrischen Komponenten gegen Wasser, das aus der Wassermatratze auslaufen könnte. Einen solchen Schutz gewährleistet grundsätzlich die die Wassermatratze umgebende Wanne, die auslaufendes Wasser auffängt. Zur Erhöhung der Sicherheit ist gemäß Anspruch 5 der Sockel mit einem seine Oberseite und die Seiten abdichtend bedeckenden Mantel versehen. Dieser Mantel kann beispielsweise aus einer Kunststoffschale bestehen.

Alternativ hierzu sind nach Anspruch 6 die elektrischen Versorgungseinrichtungen für die Strahlungsapparatur in einem separaten Schrank untergebracht, so daß sich unter der Wassermatratze keine elektrischen Teile befinden, die bei einer Zerstörung oder Beschädigung der Wassermatratze und/oder des Sockels von dem Wasser erreicht werden können.

In jedem Fall sollte derjenige Raum, in dem die elektrische Versorgungseinrichtung enthalten ist, hinreichend Bodenfreiheit gegenüber dem Fußboden haben, um im Falle des Auslaufens der Wassermatratze von dem dann vom Fußboden aus aufsteigenden Wasser nicht erreicht zu werden. In dem Raum, in dem das Bräunungsgerät enthalten ist, steigt bei auslaufender Wassermatratze das Wasser nur geringfügig an, so daß eine Bodenfreiheit von etwa 5 cm in jedem Fall ausreichen dürfte, um sicherzustellen, daß die Versorgungseinrichtungen nicht von dem aufsteigenden Wasser erreicht werden können.

Im folgenden werden unter Bezugnahme auf die Zeichnungen Ausführungsbeispiele der Erfindung näher erlautert.

Es zeigen:

Fig. 1 eine schematische perspektivische Ansicht einer ersten Ausführungsform des Bräunungsgerätes,

Fig. 2 eine schematische perspektivische Ansicht einer zweiten Ausführungsform des Bräunungsgerätes,

Fig. 3 einen Querschnitt durch Liege und Sockel bei dem Ausführungsbeispiel der Fig. 1 und

Fig. 4 einen Querschnitt durch Liege und Sockel bei dem Ausführungsbeispiel von Fig. 2.

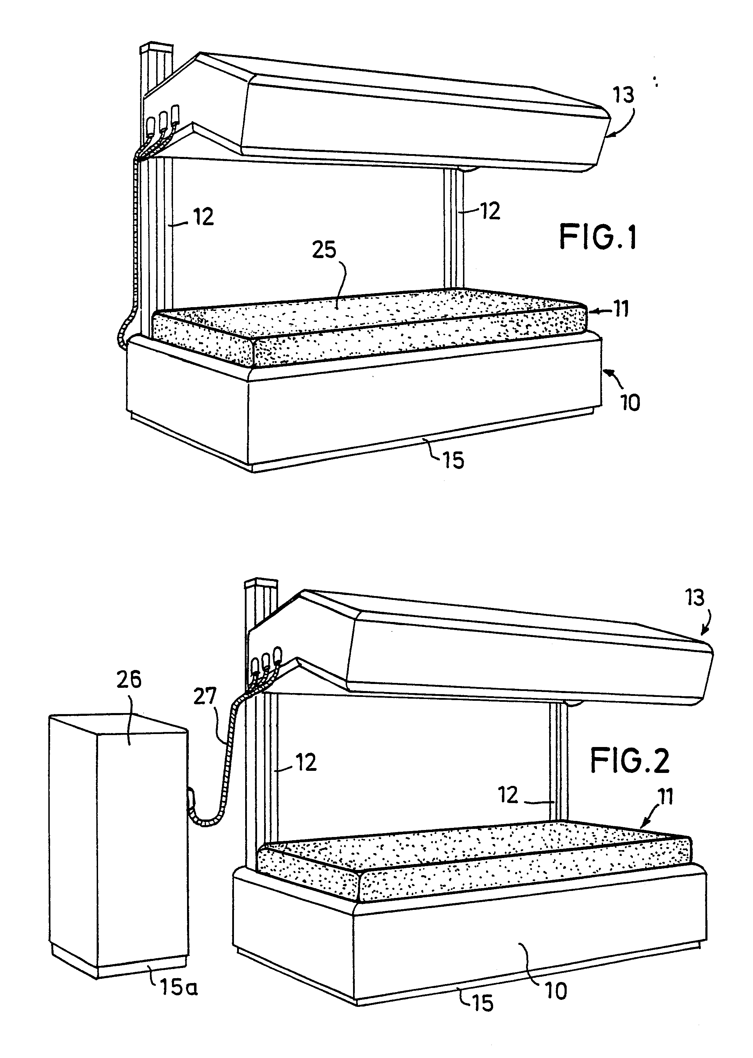

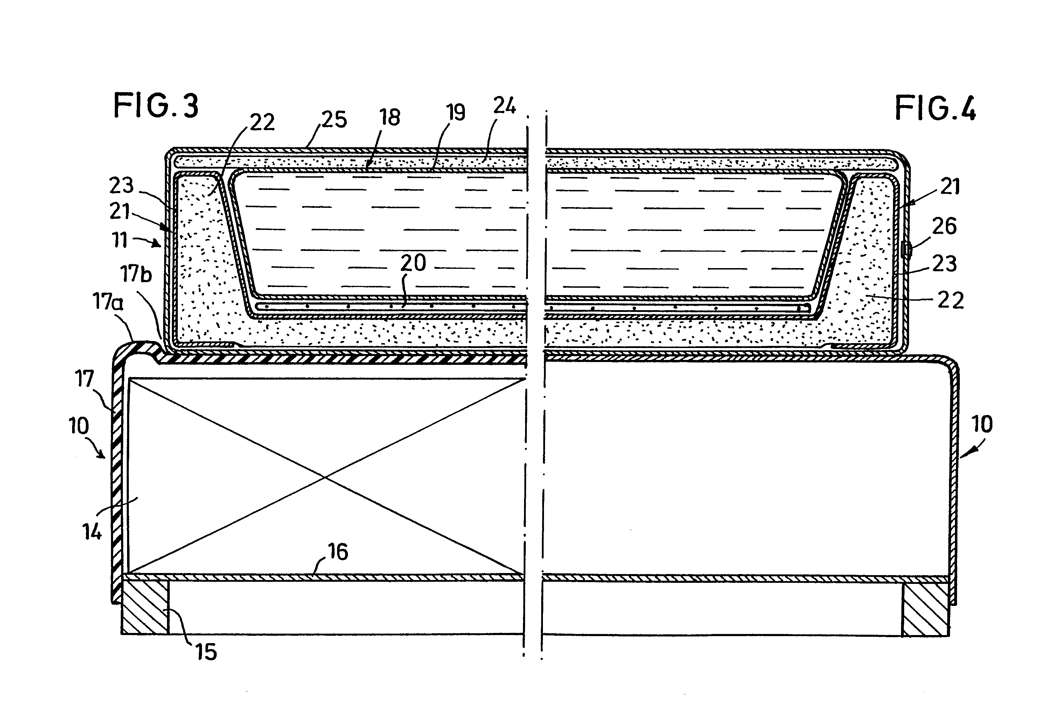

Das Bräunungsgerät nach den Fig. 1 und 3 weist einen langgestreckten kastenförmigen Sockel 10 auf, auf dem die Liege 11 angeordnet ist. Die Liege 11 hat die Größe einer üblichen Matratze, so daß ein Mensch bequem auf ihr liegen kann. Am Sockel 10 sind vertikale Ständer 12 befestigt, an denen die Strahlungsapparatur 13 höhenverstellbar angebracht ist. Die Strahlungsapparatur 13 befindet sich über der Liege 11, die sie nach Art eines Daches bedeckt. Die Ständer 12 weisen Längsführungen auf, in denen die Strahlungsapparatur 13 verfahrbar ist. Die Strahlungsapparatur 13 enthält Hochdruck-Quartzlampen, die eine nach unten gerichtete Bräunungsstrahlung erzeugen.

Fig. 3 zeigt einen Querschnitt durch Sockel und Liege nach Fig. 1. Der Sockel 10 enthält die elektrischen Umform-und Versorgungseinrichtungen 14 für die Strahlungsapparatur 13. Er weist ein Fußgestell 15 auf, das auf dem Fußboden ruht und die die Versorgungseinrichtung 14 tragende Bodenwand 16 des Sockels 10 trägt. Der Sockel 10 hat einen wasserundurchlässigen Mantel 17, der ihn an der Oberseite und an den Seitenwänden umgibt. Dieser Mantel 17 besteht aus einer bruchsicheren Kunststoffschale, die nur an ihrer Unterseite offen ist und das Fußgestell 15 auf einem Teil seiner Höhe umgibt. Der obere Rand 17a des Mantels 17 ist hochgezogen und er umgibt eine Mulde 17b zum Zentrieren der Liege 11.

Die Liege 11 enthält eine Wassermatratze 18 aus einer mit Wasser gefüllten Folie 19. Unter der Wassermatratze 18 befindet sich eine elektrische Heizvorrichtung 20. Die Wassermatratze 18 und die Heizvorrichtung 20 sind in einer Wanne 21 angeordnet und von dieser Wanne, die nur nach oben hin offen ist, allseitig umgeben. Die Wanne 21 weist einen Kern 22 aus Schaumstoff auf, welcher mit einer Schale 21 umgeben ist. Der Rand der Wanne 21 schließt etwa bündig mit der Oberseite der Wassermatratze 18 ab. Diese Oberseite ist mit einer aus Schaumstoff bestehenden Polsterschicht 24 bedeckt, die sich bis über den Rand der Wanne 21 erstreckt. Diese Polsterschicht 24 hat eine Stärke von etwa 2 cm.

Die Wassermatratze 18 ist zusammen mit der Wanne 21 und der Polsterschicht 24 von einem Überzug 25 aus textilem Stoff umgeben. Dieser Überzug kann mit einem Reißverschluß 26 versehen sein, um ausgewechselt zu werden.

Während bei dem Ausführungsbeispiel der Fig. 1 und 3 die elektrischen Versorgungseinrichtungen 14 im Sockel 10 angeordnet sind, sind sie bei dem Ausführungsbeispiel der Fig. 1 und 4 in einem separaten Schrank 26 untergebracht, der neben dem Sockel und unabhängig von diesem verschiebbar angeordnet ist. Von dem Schrank 26 führen Kabel 27 zur Strahlungsapparatur 13.

Die Liege 11 ist bei dem zweiten Ausführungsbeispiel in gleicher Weise aufgebaut wie bei dem ersten Ausführungsbeispiel. Der Sockel 10 braucht jedoch keinen wasserundurchlässigen Mantel aufzuweisen, da er lediglich die Funktion einer Stützstruktur für die Liege hat. Der die Versorgungseinrichtungen enthaltende Schrank 26 ist mit einem Fußgestell 15a versehen, das die erforderliche Bodenfreiheit der Versorgungseinrichtungen zum Schutz vor aufsteigendem Wasser gewährleistet.

## Ansprüche

1. Bräunungsgerät mit einer Liege (11) an der mindestens ein Ständer (12) für eine über der Liege angeordnete Strahlungsapparatur (13) befestigt ist,

**dadurch gekennzeichnet,** daß die Liege (11) eine Wassermatratze (18) aufweist.

2. Bräunungsgerät nach Anspruch 1, dadurch gekennzeichnet, daß die Wassermatratze (18) in einer Wanne (21) angeordnet ist, die einen mit Wasser undurchlässigem Material (23) überzogenenen Schaumstoffkern (22) aufweist.

3. Bräunungsgerät nach Anspruch 2, dadurch gekennzeichnet, daß die Wanne (21) und die Wassermatratze (18) mit einer Polsterschicht (24) bedeckt sind.

4. Bräunungsgerät nach Anspruch 2 oder 3, dadurch gekennzeichnet, daß die Wanne (21) zusammen mit der Wassermatratze (18) in einem auswechselbaren Überzug (25) enthalten ist.

5. Bräunungsgerät nach einem der Ansprüche 1 bis 4, dadurch gekennzeichnet, daß die Liege (11) auf einem Sockel (10) angeordnet ist, der elektrische Versorgungseinrichtungen (14) für die Strahlungsapparatur (13) enthält, und daß der Sockel (10) mit einem seine Oberseite und die Seiten abdichtend bedeckenden Mantel (17) versehen ist.

6. Bräunungsgerät nach einem der Ansprüche 1 bis 4, dadurch gekennzeichnet, daß die Liege (11) auf einem Sockel (10) angeordnet ist, der keine elektrischen Bauteile enthält und daß die elektrischen Versorgungseinrichtungen für die Strahlungsapparatur (13) in einem separaten Schrank (26) untergebracht sind, der durch Kabel (27) mit der Strahlungsapparatur (13) verbunden ist.

7. Bräunungsgerät nach Anspruch 5 oder 6, dadurch gekennzeichnet, daß der Sockel (10) bzw. der Schrank (26) auf einem Fußgestell (15, 15a) mit mindestens etwa 5 cm Bodenfreiheit angeordnet ist.

13

12

25

12

**FIG.1**

11

10

15

13

26

27

12

**FIG.2**

12

11

15a

15

10

FIG.3    22   25   18   19   24    FIG.4

23
21
11

17b
17a

17
10

14

15

20

21
26
23
22

10

16

0 280 062

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 113 832 (REY) <br> * Seite 4, Zeile 7 - Seite 6, Zeile 23; Figuren * | 1,3 | A 61 N 5/06 <br> A 47 C 27/08 |
| A | | 2,5 | |
| | --- | | |
| X | DE-A-2 910 468 (MUTZHAS) <br> * Seite 8, Zeile 29 - Seite 9, Zeile 16 * | 1,5 | |
| | --- | | |
| A | US-A-4 187 566 (PETERSON) <br> * Spalte 3, Zeile 3 - Spalte 4, Zeile 60 * | 2,3,4 | |
| | --- | | |
| A | US-A-4 354 289 (RICHARDS) <br> * Spalte 2, Zeile 11 - Spalte 3, Zeile 42 * | 2,4,5,7 | |
| | ----- | | |

**RECHERCHIERTE SACHGEBIETE (Int. Cl.4)**

A 61 N
A 47 C

Der vorliegende Recherchenbericht wurde für alle Patentansprüche erstellt

| Recherchenort | Abschlußdatum der Recherche | Prüfer |
|---|---|---|
| DEN HAAG | 11-05-1988 | LEMERCIER D.L.L. |